# FASCICULE DE BREVET EUROPEEN

(11) **EP 0 455 682 B1**
(45) Date de publication et mention de la délivrance du brevet: **29.12.1993**
(21) Numéro de dépôt: 90902296.4
(22) Date de dépôt: 25.01.1990
(51) Int. Cl.: C07K 7/10, C12N 15/12, C12P 21/02, C12N 15/00

(54) **POLYPEPTIDES AYANT UNE ACTIVITE DE RECEPTEUR BETA-ADRENERGIQUE CHEZ L'HOMME, IMPLIQUES DANS LA REPONSE LIPOLYTIQUE, ACIDES NUCLEIQUES CODANT POUR CES POLYPEPTIDES ET UTILISATION DE CES POLYPEPTIDES POUR LE CRIBLAGE DE SUBSTANCE ACTIVE SUR CES POLYPEPTIDES**
POLYPEPTIDE MIT EINER AKTIVITÄT DES MENSCHLICHEN BETA-ANDRENERGISCHEN REZEPTORS, EINBEZOGEN IN DIE LIPOLYTISCHE ANTWORT, DEREN NUKLEINSÄURESEQUENZEN
POLYPEPTIDES HAVING A HUMAN BETA-ADRENERGIC RECEPTOR ACTIVITY, AND PLAYING A ROLE IN LIPOLYTIC RESPONSE, NUCLEIC ACIDS CODING FOR THESE POLYPEPTIDES AND USE OF SAID POLYPEPTIDES FOR SCREENING SUBSTANCES WHICH ACT ON THESE POLYPEPTIDES

(30) Priorité: 25.01.1989 FR 8900918
(43) Date de publication de la demande: 13.11.1991
(73) Titulaire: CENTRE NATIONAL DE LA RECHERCHE SCIENTIFIQUE, 75007 Paris (FR)
(72) Inventeur: EMORINE, Laurent, F-75013 Paris (FR); MARULLO, Stefano, F-75013 Paris (FR); STROSBERG, Donny, F-75015 Paris (FR)
(74) Mandataire: Gutmann, Ernest
(86) Numéro de dépôt international: FR9000054
(87) Numéro de publication internationale: WO9008775

(56) Documents cités:
- The Journal of Biological Chemistry, volume 262, No. 15, 25 May 1987, American Society of Biological Chemists, Inc. (US), B.K. Kobilka et al.: "Delineation of the intronless nature of the genes for the human and hamster beta2-adrenergic receptor and their putative promoter regions", pages 7321-7327
- FEBS Letters, volume 211, No. 2, 26 January 1987, Elsevier Science Publishers B.V. (Amsterdam, NL), F.-Z. Chung et al.: "Cloning and sequence analysis of the human brain beta-adrenergic receptor. Evolutionary relationship to rodent and avian beta-receptors and porcine muscarinic receptor, pages 200-206
- Proceedings of the National Academy of Sciences of the USA, vol. 85, no. 19, octobre 1988, (Washington, DC, US), S. Cotecchia et al.: "Molecular cloning and expression of the cDNA for the hamster alphal-adrenergic receptor", pages 7159-7163
- Science, vol. 238, no. 4827, 30 octobre 1987, (Washington, DC, US), B.K. Kobilka et al.: "Cloning, sequencing, and expression of the gene alpha2-adrenergic receptor", pages 650-655

## Description

L'invention a pour objet des polypeptides ayant une activité de récepteur β-adrénergique chez l'homme, et plus particulièrement impliqués dans la réponse lipolytique des tissus adipeux, et les gènes codant pour ces polypeptides.

L'invention est également relative
- à des vecteurs contenant les gènes codant pour des polypeptides ayant une activité β-adrénergique,
- à des hôtes cellulaires transformés par des gènes codant pour les susdits polypeptides,
- à des sondes nucléotidiques suceptibles de s'hybrider avec les gènes codant pour les susdits polypeptides,
- à des anticorps polyclonaux et monoclonaux dirigés contre les susdits polypeptides et utilisables dans un but de diagnostic in vitro,
- à des trousses (ou kits) pour étudier le degré d'affinité de certaines susbtances pour les susdits polypeptides ,
- à des médicaments contenant des substances actives sur des susdits polypeptides ayant une activité de récepteur β-adrénergique, et plus particulièrement destinés au traitement de l'obésité, du diabète et de l'hyperlipidémie.

Les catécholamines telles que l'adrénaline et la noradrénaline, les agonistes synthétiques de ces catécholamines, qui miment leurs fonctions biologiques et les antagonistes, qui bloquent ces fonctions, exercent leurs effets en se liant à des sites de reconnaissance (récepteurs adrénergiques) spécifiques situés sur les membranes cellulaires.

Deux classes principales de récepteurs adrénergiques ont été définies, les récepteurs adrénergiques α et les récepteurs adrénergiques β.

Dans l'ensemble de ces deux classes, on distingue quatre sous-types de ces récepteurs aux catécholamines (α1, α2, β1 et β2-AR). Leurs gènes ont été récemment isolés et identifiés (5-8). L'analyse de ces gènes a permis de reconnaître leur appartenance à une famille de récepteurs membranaires intégraux présentant certaines homologies (9-10), notamment au niveau de 7 régions transmembranaires. Celles-ci sont couplées à des protéines régulatrices, appelées protéines G, susceptibles de fixer des molécules de guanosine triphosphate (GTP).

Plus précisément, les protéines G sont des protéines ayant la capacité de s'interposer structuralement et fonctionnellement entre des récepteurs et des enzymes catalysant la production de médiateurs intracellulaires (tels que l'adénylate cyclase, la guanylate cyclase, les phospholipases, les kinases) ou entre des récepteurs et des canaux ioniques dont l'ouverture contrôlée entraîne un flux d'ions (tels que les ions calcium, potassium, sodium, hydrogène) dans la cellule.

Ces protéines ont des fonctions de transduction et de couplage.

La susdite famille de récepteurs est désignée par "famille R₇G" (10). Elle comprend notamment les récepteurs muscariniques de l'acétylcholine, les récepteurs de la serotonine, les récepteurs des neuropeptides, substance K et angiotensine II et les récepteurs visuels de la famille des opsines (9-10).

Jusqu'à une époque très récente, la définition des sous-types de récepteurs s'appuyait principalement sur l'analyse, dans les systèmes hétérogènes, des propriétés physiologiques et de liaison de différents ligands. Dans la famille R₇G, plusieurs gènes codant pour des sous-types de récepteurs définis par leurs propriétés pharmacologiques ont été clonés et caractérisés. Des sondes obtenues à partir de ces gènes ont permis d'identifier des sous-types de récepteurs supplémentaires (11-12).

La nature exacte du récepteur adrénergique β, susceptible de moduler les fonctions physiologiques telles que la thermogénèse dans les cellules adipeuses, ainsi que la relaxation intestinale est restée obscure. En rapport avec cette dernière propriété, on a trouvé que l'isoproterenol inhibe toujours les contractions de l'ileum de cobaye induite par voie cholinergique (3), en dépit du blocage total des récepteurs adrénergiques connus du type α et β avec la pentholamine et le propanolol.

En procédant à une étude détaillée des effets physiologiques des agonistes et de l'inhibition par les antagonistes des polypeptides ayant une activité de récepteur β adrénergique (1-3), a été émise l'hypothèse de l'existence d'un nouveau sous-type de récepteur β adrénergique.

Cette hypothèse a été remise en cause par une hypothèse contradictoire, résultant de l'analyse du contenu en récepteur β d'un tissu adipeux par des études de liaison. Cette analyse a conduit à la conclusion - qui constitue l'état de la technique le plus récent - que les récepteurs β adrénergiques lipolytiques sont uniquement du sous-type β1 (13).

Par ailleurs, parmi les composés utilisés en pharmacopée moderne, une position dominante est occupée par les agonistes ou antagonistes β-adrénergiques (β1 ou β2-AR). Les médicaments disponibles peuvent, en dépit de leur remarquable efficacité, produire des effets secondaires, potentiellement dus à l'interaction avec d'autres récepteurs homologues.

L'invention lève l'incertitude en faveur de l'hypothèse antérieurement formulée, mais ultérieurement écartée de l'existence de polypeptides ayant une activité de récepteur β adrénergique autre que celle des récepteurs β1 et β2. Elle donne en effet accès à de nouveaux polypeptides ayant une activité de récepteur β adrénergique, ne s'apparentant ni à celle des récepteurs adrénergiques β1, ni à celle des récepteurs adrénergiques β2.

L'invention a également pour objet des procédés de criblage de nouveaux médicaments agissant sur les nouveaux polypeptides ayant une action de récepteur β-adrénergique et destinés entre autre au traitement de l'obésité, du diabète gras et du diabète des sujets non insulino-dépendants, ainsi qu'au traitement des hyperlipidémies.

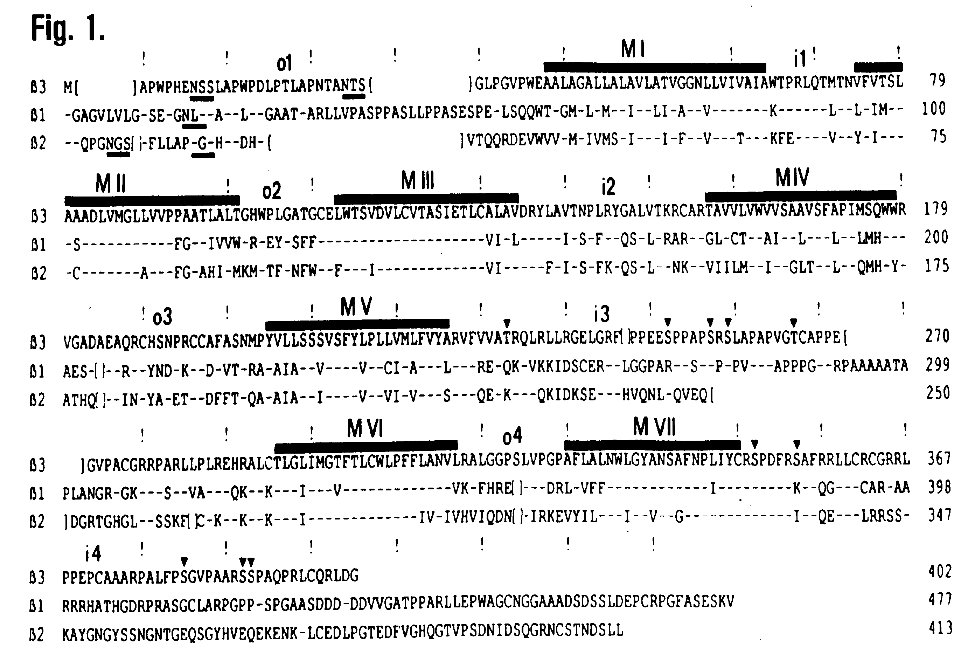

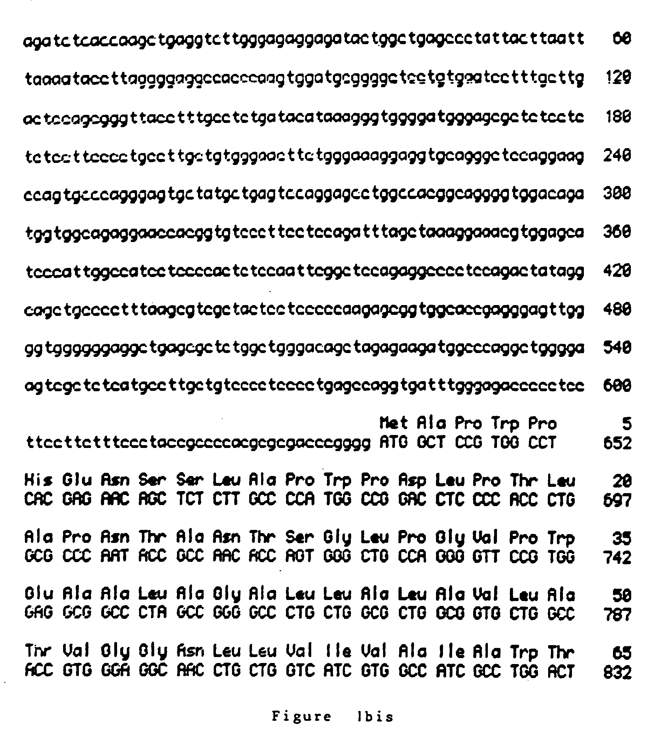

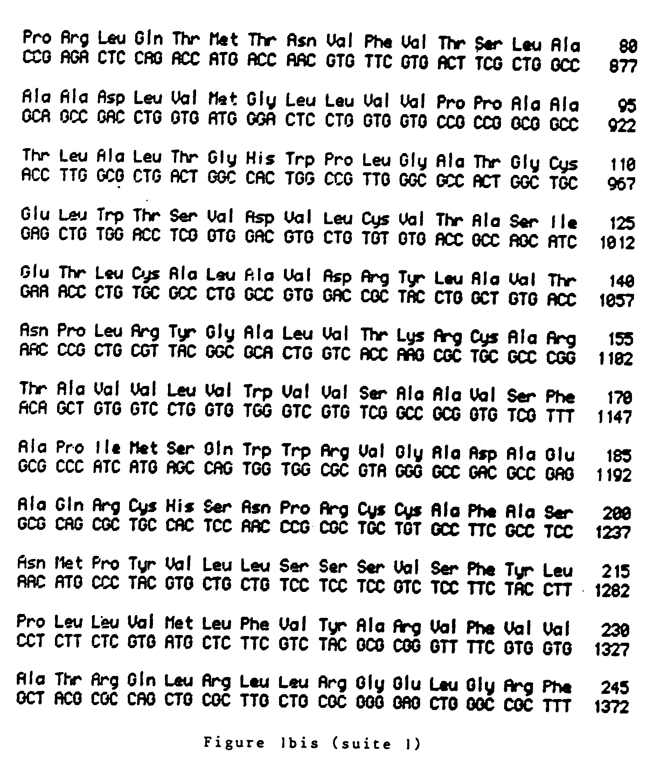

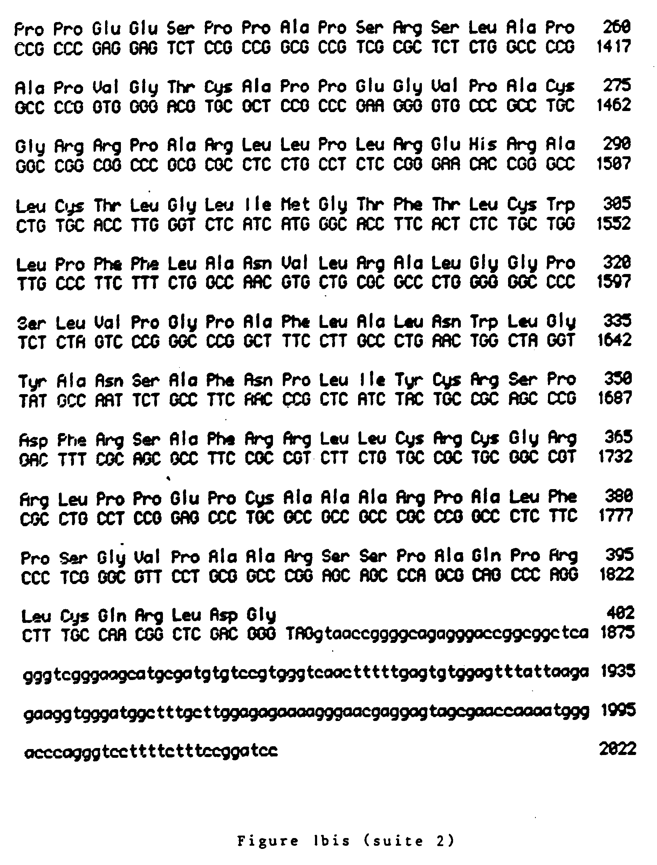

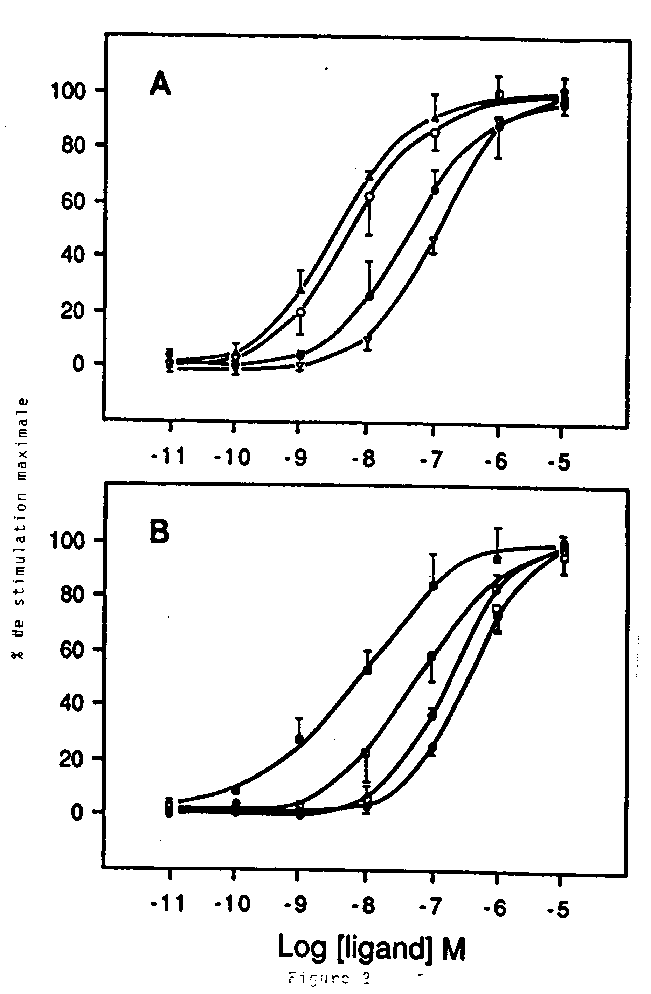

En particulier, le nouveau polypeptide de l'invention ayant une activité de récepteur β-adrénergique :
- contient la séquence de 402 acides aminés de la Figure 1 ou
un fragment de cette séquence, ce fragment étant tel que
* soit il contient néanmoins les sites contenus dans cette séquence et dont la présence est nécessaire pour que, lorsque ce fragment est exposé à la surface d'une cellule, il soit capable de participer à l'activation de l'adénylate cyclase en présence d'un agoniste, cette activation étant croissante dans l'ordre des agonistes suivants : salbutamol, BRL 28410, BRL 37344 et (1)-isoproterenol,
* soit il est susceptible d'être reconnu par des anticorps qui reconnaissent également la susdite séquence de 402 acides aminés, mais ne reconnaissent ni le récepteur adrénergique β1, ni le récepteur adrénergique β2
* soit il est susceptible de générer des anticorps qui reconnaissent la susdite séquence de 204 acides aminés mais ne reconnaissent ni le récepteur β1, ni le récepteur adrénergique β2.

La reconnaissance de la susdite séquence de 402 acides aminés par les susdits anticorps - ou du susdit fragment par les susdits anticorps - signifie que la susdite séquence forme un complexe avec l'un des susdits anticorps.

La formation du complexe antigène (c'est-à-dire séquence de 402 acides aminés ou susdit fragment)-anticorps et la détection de l'existence d'un complexe formé peuvent se faire de la façon suivante:
- on laisse incuber l'antigène et l'anticorps pendant 1h à température ambiante, puis pendant une nuit à 4°C, en tampon PLT;
- le tampon PLT ayant la composition suivante : 10 mM de phosphate de sodium, 145 mM de NaCl, 5% de lait écrémé lyophilisé (p/v), 0.1% de Tween 20 (v/v), le pH étant de 7.4,
- on effectue ensuite quatre lavages de 5 min. avec le tampon PLT,
- on laisse incuber un deuxième anticorps biotynilé 1h à température ambiante en tampon PLT,
- on effectue quatre lavages de 5 min. en tampon PLT,
- on laisse incuber la streptavidine peroxydase, 1h à température ambiante en tampon PLT,
- on procède à quatre lavages en tampon PLT puis
- on effectue un lavage dans le milieu constitué par: 10 mM de phosphate de sodium, 145 mM de NaCl, 0.1% de Triton x 100, le pH étant de 7.4,
- on effectue un nouveau lavage dans le milieu ayant la composition indiquée ci-dessus, mais ne contenant pas de Triton x 100, puis
- on révèle le complexe antigène-anticorps formé par des techniques classiques.

Pour ce qui est de la définition des "fragments contenant les sites" et répondant à la susdite définition, on se reportera ci-après dans la description.

Les nouveaux récepteurs β-adrénergiques de l'invention sont donc caractérisés par des propriétés différentes de celles des récepteurs β1 et β2, en ce qu'ils se comportent de façon différente vis-à-vis de substances respectivement antagonistes et agonistes vis-à-vis de β1 et β2.

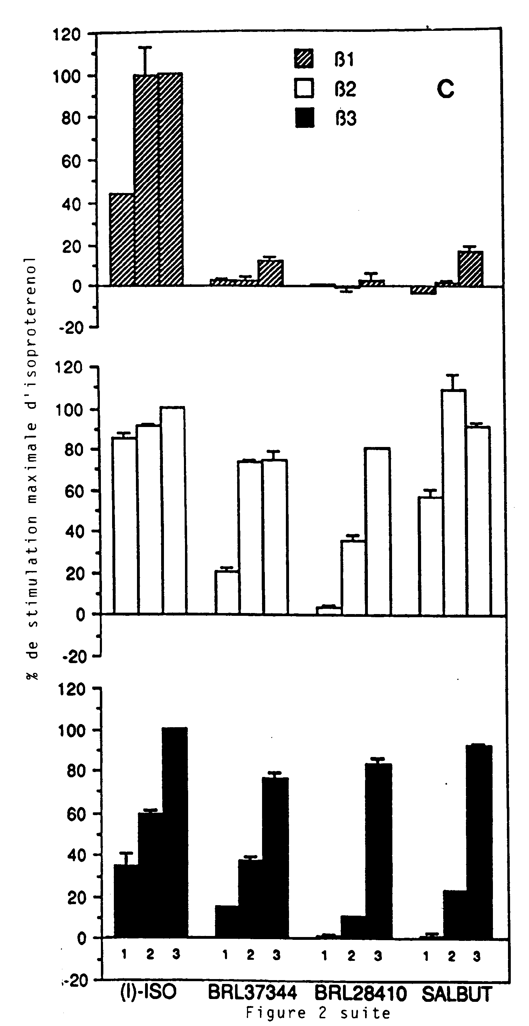

La détermination de l'ordre dans lequel les polypeptides de l'invention participent à l'activation de l'adénylate cyclase en présence des agonistes respectivement présents : salbutamol, BRL 28410, BRL 37344 et (1)-isoproterenol, est décrite dans le commentaire relatif à la Figure 2C, qui fait suite à la présente description.

L'ordre indiqué ci-dessus est totalement différent de celui obtenu avec les mêmes agonistes, lors des réponses médiatisées par l'intermédiaire des récepteurs adrénergiques β1 ou β2.

Pour des cellules "CHO-β3" définies ci-après, cet ordre est voisin de celui déterminé pour la stimulation de la lipolyse dans des cellules adipeuses de rat (1). La seule différence est constituée par l'ordre relatif entre l'isoproterenol et BRL 37344. Cependant, l'isoproterenol dont il est question dans l'invention est l'isomère lévogyre de l'isoproterenol alors que celui de la référence (1) est le mélange racémique (dl).

Le BRL 28410 est défini dans la référence (1).

Le BRL 37344 est défini dans la référence (1).

L'invention concerne également des protéines chimères dans lesquelles le polypeptide tel que défini plus haut ou des parties de celui-ci sont réunis à un enchaînement d'acides aminés hétérologue par rapport à ce polypeptide.

Des protéines chimères avantageuses de l'invention sont constituées par celles dans lesquelles l'enchaînement d'acides aminés hétérologue est choisi parmi la séquence du récepteur adrénergique β1 ou un fragment de ce récepteur ou la séquence du récepteur adrénergique β2 ou un fragment de ce récepteur.

Les polypeptides et protéines chimères de l'invention peuvent être glycosylés et peuvent comporter ou non des ponts disulfure.

Dans la suite de la description, les polypeptides de l'invention seront, pour simplifier, désignés par "récepteurs β3".

Les récepteurs β3 de l'invention sont également tels que la capacité à stimuler l'adenylate cyclase et/ou l'accumulation de l'AMP cyclique induite par l'isoproterenol, n'est pas inhibée par les composés suivants :
- practolol,
- nadolol,
- CGP 12,177,
- butoxamine,
- alprenolol,
- propanolol,
- pindolol,
- oxprenolol,

utilisés à des concentrations inférieures ou égales à 10⁻⁴M, dans les conditions suivantes :
. en ce qui concerne l'accumulation de l'AMP cyclique, des cellules sont cultivées et récoltées après traitement avec Versene/EDTA (Eurobio, Paris), lavées et resuspendues dans un milieu de Hank contenant 20 mM d'Hepes tamponnés à pH 7.4, de l'acide ascorbique 1 mM et de l'isobutyl-méthyle-xanthine 0.1mM; des aliquotes de 10⁶ cellules sont incubées pendant 30 minutes, avec les inhibiteurs à 10⁻⁴M avant l'addition de 5.10⁻⁹M d'isoproterenol ; l'incubation est poursuivie encore pendant 30 minutes, et on mesure les niveaux d'AMP cyclique, par exemple selon les spécifications de la trousse d'essais Amersham.

La stimulation de l'adenylate cyclase par les récepteurs β3 de l'invention, ainsi que la mesure de l'accumulation de cAMP peuvent être effectuées selon des méthodes classiques.

Le CGP12,177 est un produit fabriqué par la société CIBA-GEIGY.

Les conditions utilisées sont semblables à celles dans lesquelles les susdits composés se comportent comme des antagonistes vis-à-vis des récepteurs adrénergiques β1 et β2.

Les récepteurs β3 de l'invention sont également tels que le pindolol et l'oxprenolol favorisent l'accumulation de l'AMP cyclique dans des cellules CHO transfectées avec le susdit récepteur β3 , dans les conditions suivantes :
. en ce qui concerne l'accumulation de l'AMP cyclique, des cellules sont cultivées et récoltées après traitement avec Versene/EDTA (Eurobio, Paris), lavées et resuspendues dans un milieu de Hank contenant 20 mM d'Hepes tamponnés à pH 7.4, de l'acide ascorbique 1 mM et de l'isobutyl-méthyle-xanthine 0.1 mM; des aliquotes de 10⁶ cellules sont incubées pendant 30 minutes, dans un volume total de 1 ml avec des concentrations de pindolol ou d'oxprenolol supérieures à 10⁻¹¹M et les niveaux de cAMP sont mesurés par exemple selon les spécifications de la trousse d'essai Amersham.

Un récepteur β3 avantageux de l'invention est constitué par l'enchaînement des acides aminés 1 à 402, représentés sur la Figure 1.

Ce récepteur β3 est considéré comme comportant sept régions transmembranaires hydrophobes séparées par des boucles hydrophiles intra et extracellulaires.

L'invention concerne également les polypeptides variants qui correspondent aux polypeptides sus-définis comportant certaines mutations localisées, sans que les polypeptides ne perdent les propriétés de récepteur β3-adrénergique. Parmi ces variants, on peut mentionner ceux qui sont reconnus par des anticorps reconnaissant les régions transmembranaires, ainsi que ceux qui sont reconnus par des anticorps reconnaissant les régions autres que les régions transmembranaires.

L'invention concerne également des acides nucléiques qui comprennent ou qui sont constitués par un enchainement de nucléotides codant pour l'un quelconque des récepteurs β3 précédemment définis.

Plus particulièrement, l'invention concerne l'acide nucléique qui comprend l'enchaînement de nucléotides représenté sur la Figure 1bis, s'étendant de l'extrémité constituée par le nucléotide en position 1 à celle constituée par le nucléotide en position 2022.

L'invention concerne également l'acide nucléique représenté sur la Figure 1bis comprenant ou étant constitué par l'enchaînement s'étendant de l'extrémité constituée par le nucléotide en position 638 à celle constituée par le nucléotide en position 1843.

L'acide nucléique sus-défini correspond à la partie codante du gène correspondant au polypeptide représenté sur la Figure 1.

Font également partie de l'invention les acides nucléiques variants par rapport à ceux sus-définis et qui comportent certaines mutations localisées dans la mesure où ces acides nucléiques variants s'hybrident avec les acides nucléiques précédemment définis ou avec les sondes nucléiques définies ci-après dans les conditions d'hybridation définies ci-après dans la description.

Les acides nucléiques de l'invention peuvent être préparés soit par un procédé chimique, soit par d'autres procédés.

Un mode de préparation approprié des acides nucléiques (comportant au maximum 200 nucléotides - ou pb, lorsqu'il s'agit d'acides nucléiques bicaténaires) de l'invention par voie chimique comprend les étapes suivantes :
- la synthèse d'ADN en utilisant la méthode automatisée des β-cyanéthyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides - ou pb (lorsqu'il s'agit d'acides nucléiques bicaténaires) comprend les étapes suivantes:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

Un autre procédé de préparation des acides nucléiques de l'invention à partir d'ARNm comprend les étapes suivantes :
- préparation d'ARN cellulaire à partir de tout tissu exprimant le récepteur β3-adrénergique, en particulier les tissus adipeux, musculaires, hépatiques et intestinaux, selon les techniques décrites par Maniatis et al. dans "Molecular cloning", Cold Spring Harbor Laboratory, 1982, et Ausubel F.M., Brent R., Kingston R.E., Moore D.D., Smith J.A., Seidman J.G. et Struhl K. (1989) Current Protocols in Molecular Biology, chapitre 4, Greene Publishing Associates et Wiley-Interscience, New York,
- récupération et purification des ARNm par passage des ARN cellulaires totaux par chromatographie avec un oligodT immobilisé,
- synthèse d'un brin d'ADNc à partir des ARNm purifiés d'après la technique décrite dans Gene 25:263, 1983,
- clonage des acides nucléiques ainsi obtenus dans un vecteur plasmidien approprié et récupération de la séquence nucléotidique recherchée en utilisant une sonde d'hybridation appropriée.

Pour préparer les acides nucléiques de l'invention, les sondes d'hybridation oligonucléotidiques synthétisées chimiquement sont les suivantes :
. - celle définie par la séquence d'acides nucléiques représentée sur la Figure 1bis, s'étendant de l'extrémité constituée par le nucléotide en position 1, à celle constituée par le nucléotide en position 637
. - celle définie par la séquence d'acides nucléiques représentée sur la Figure 1bis, s'étendant de l'extrémité constituée par le nucléotide en position 638 à celle constituée par le nucléotide en position 745,
. - celle définie par la séquence d'acides nucléiques représentée sur la Figure 1bis, s'étendant de l'extrémité constituée par le nucléotide en position 1313, à celle constituée par le nucléotide en position 1513,
. - celle définie par la séquence d'acides nucléiques représentée sur la Figure 1bis, s'étendant de l'extrémité constituée par le nucléotide en position 1679, à celle constituée par le nucléotide en position 1843,
. - celle définie par la séquence d'acides nucléiques représentée sur la Figure 1bis, s'étendant de l'extrémité constituée par le nucléotide en position 1844, à celle constituée par le nucléotide en position 2022,
. - celle définie par la séquence d'acides nucléiques représentée sur la Figure 1bis, s'étendant de l'extrémité constituée par le nucléotide en position 638, à celle constituée par le nucléotide en position 1843.

ou leur séquence nucléotidique complémentaire;
ce sont des séquences qui ont été dérivées de celles de la Figure 1bis et elles peuvent être utilisées dans les conditions d'hybridation décrites par Maniatis et al. dans "Molecular cloning", Cold Spring Harbor Laboratory, 1982.

La synthèse du brin d'ADNc et son amplification subséquente in vitro peut également être effectuée en utilisant la méthode PCR (Polymerase Chain Reaction), comme décrit par exemple par Goblet et al. dans Nucleic Acid Research, 17, 2144, 1989 "One step amplification of transcripts in total RNA using Polymerase Chain Reaction", en utilisant deux amplimères chimiquement synthétisés définis à partir de la séquence de la Figure 1bis. Des amplimères appropriés sont par exemple : celui défini sur la Figure 1bis du nucléotide 638 au nucléotide 667 et celui défini sur la Figure 1bis du nucléotide 1815 au nucléotide 1843.

Le fragment d'acides nucléiques amplifié peut être ensuite cloné selon les techniques décrites dans Ausubel F.M., Brent R., Kingston R.E., Moore D.D., Smith J.A., Seidman J.G. et Struhl K. (1989) Current Protocols in Molecular Biology, chapitre 3, Greene Publishing Associates et Wiley-Interscience, New York.

L'invention concerne également les vecteurs recombinants, en particulier pour le clonage et/ou l'expression, notamment du type plasmide, cosmide ou phage, contenant un acide nucléique de l'invention en l'un de ses sites non essentiels pour sa réplication.

L'invention concerne également le vecteur M13mp18-Huβ3, (n°1085), constitué par un bactériophage déposé sous le n°I-833 le 20 janvier 1989 à la CNCM, 25 rue du Docteur Roux, Paris.

Un vecteur approprié de l'invention, contient en l'un de ses sites non essentiels pour sa réplication des éléments nécessaires pour promouvoir l'expression d'un polypeptide selon l'invention, dans un hôte cellulaire et éventuellement un promoteur reconnu par les polymérases de l'hôte cellulaire, en particulier un promoteur inductible et éventuellement une séquence signal et une séquence d'ancrage.

L'invention concerne également un hôte cellulaire transformé par un vecteur recombinant défini précédemment comprenant les éléments de régulation permettant l'expression de la séquence nucléotidique codant pour l'un des polypeptides selon l'invention dans cet hôte.

Par hôte cellulaire on entend tout organisme susceptible d'être maintenu en culture.

L'un des microorganismes utilisés peut être constitué par une bactérie, notamment Escherichia Coli.

Un organisme de choix est constitué par un organisme eucaryotes tel que des cellules CHO (Chinese Hamster Ovary).

Mais d'autres organismes peuvent être utilisés tout aussi aisément, naturellement sous réserve que l'on dispose pour chacun d'entre eux des vecteurs, notamment plasmidiques, susceptibles de s'y répliquer et des séquences de nucléotides insérables dans ces vecteurs et capables, lorsqu'elles sont suivies dans ces vecteurs par un insérat codant pour un polypeptide de l'invention, d'assurer l'expression de cet insérat dans les organismes choisis et leur transport dans les membranes de ces hôtes cellulaires.

L'invention concerne également les anticorps dirigés de façon spécifique contre l'un des polypeptides de l'invention, ces anticorps étant tels qu'ils ne reconnaissent ni le récepteur adrénergique β1, ni le récepteur adrénergique β2. En particulier, ces anticorps reconnaissent les séquences d'acides aminés suivantes :
1 à 36 178 à 201
64 à 74 223 à 291
101 à 108 314 à 325
133 à 135 345 à 402

Pour obtenir les anticorps, on peut injecter chez l'animal l'un de susdits polypeptides.

On prépare des anticorps monoclonaux par fusion cellulaire entre des cellules de myélome et des cellules spléniques de souris immunisées, selon les procédés classiques.

L'invention concerne également les sondes nucléotidiques synthétiques ou non, s'hybridant avec l'un des acides nucléiques définis ci-dessus ou leurs séquences complémentaires ou leur ARN correspondant, ces sondes étant telles qu'elles s'hybrident ni avec le gène ou l'ARN messager des récepteurs β1 et β2 adrénergiques.

Les sondes de l'invention comportent au minimum 10, avantageusement 15 acides nucléiques et peuvent comporter au maximum la totalité de la séquence nucléotidique représentée sur la figure 1bis.

Pour les sondes les plus courtes, c'est-à-dire d'environ 10 à environ 100 nucléotides, des conditions d'hybridation appropriées sont les suivantes:
750 mM de NaCl, 75 mM de Tri-sodium citrate, 50 µg/ml d'ADN de sperme de saumon, 50 mM de phosphate de sodium, 1 mM de pyrophosphate de sodium, 100µM d'ATP, 10 à 25% de formamide, 1% Ficoll (Pharmacia poids moléculaire moyen de 400,00), 1% de polyvinylpyrrolidone, 1% de serum albumine bovine - pendant 14 à 16 h à 42°C.

Pour les sondes les plus longues, c'est-à-dire présentant plus d'environ 100 nucléotides, des conditions d'hybridation appropriées sont celles indiquées précédemment pour les sondes les plus courtes, mais dans lesquelles le milieu sus-défini contient 40% de formamide au lieu de 10 à 25% de formamide et contient en plus 10% de sulfate de dextrane.

L'invention concerne en particulier les sondes nucléotidiques suivantes :
. - celle définie par la séquence d'acides nucléiques représentée sur la Figure 1bis, s'étendant de l'extrémité constituée par le nucléotide en position 1, à celle constituée par le nucléotide en position 637
. - celle définie par la séquence d'acides nucléiques représentée sur la Figure 1bis, s'étendant de l'extrémité constituée par le nucléotide en position 638 à celle constituée par le nucléotide en position 745,
. - celle définie par la séquence d'acides nucléiques représentée sur la Figure 1bis, s'étendant de l'extrémité constituée par le nucléotide en position 1313, à celle constituée par le nucléotide en position 1513,
. - celle définie par la séquence d'acides nucléiques représentée sur la Figure 1bis, s'étendant de l'extrémité constituée par le nucléotide en position 1679, à celle constituée par le nucléotide en position 1843,
. - celle définie par la séquence d'acides nucléiques représentée sur la Figure 1bis, s'étendant de l'extrémité constituée par le nucléotide en position 1844, à celle constituée par le nucléotide en position 2022,
. - celle définie par la séquence d'acides nucléiques représentée sur la Figure 1bis, s'étendant de l'extrémité constituée par le nucléotide en position 638, à celle constituée par le nucléotide en position 1843.

Les polypeptides de l'invention peuvent être préparés par culture dans un milieu approprié d'un hôte cellulaire préalablement transformé par un vecteur recombinant contenant l'un des acides nucléiques définis précédemment et par récupération à partir de la susdite culture du polypeptide produit par ledit hôte cellulaire transformé.

Un autre procédé de préparation des polypeptides de l'invention est caractérisé en ce que, partant de préférence de l'amino acide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amines de l'un et carboxyle de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

S'agissant de réaliser l'expression des récepteurs adrénergiques β3 dans une bactérie, telle que E. coli ou dans une cellule eucaryote telle qu'une cellule CHO, on effectue les étapes suivantes :
- la transformation d'un hôte cellulaire compétent avec un vecteur, notamment un plasmide ou un phage, dans lequel a auparavant été insérée une séquence de nucléotides codant pour le récepteur β3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur β3 exprimé vers la membrane de façon telle que les séquences transmembranaires du récepteur β3 soient exposées à la surface de l'hôte cellulaire transformé.

S'agissant de l'expression en cellules eucaryotes, les éléments de régulation peuvent comporter le promoteur endogène des récepteurs adrénergiques ou des promoteurs viraux tels que ceux des virus SV40 ou du virus du sarcom de Roux (RSV).

S'agissant de l'expression dans E. coli, les éléments de régulation peuvent comporter le promoteur de l'opéron lactose ou de l'opéron tryptophane.

L'invention concerne également un procédé de détection de la capacité d'une molécule à se comporter comme ligand vis-à-vis d'un polypeptide de l'invention, lequel procédé comprend :
- la mise en contact de la molécule avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avèrerait effectivement posséder une affinité pour ce polypeptide,
- la détection de la formation éventuelle d'un complexe du type ligand-polypeptide.

L'invention concerne également un procédé pour l'étude de l'affinité d'un polypeptide de l'invention pour un ou plusieurs ligands déterminés, lequel procédé comprend :
- la transformation d'un hôte cellulaire compétent avec un vecteur, notamment un plasmide ou un phage, dans lequel avait auparavant été insérée une séquence de nucléotides codant pour le récepteur β3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur β3 exprimé vers la membrane de façon telle que les séquences transmembranaires du récepteur β3 soient exposées à la surface de l'hôte cellulaire transformé,
- la mise en contact de cet hôte cellulaire avec ces ligands déterminés,
- la détection d'une réaction affine entre ledit hôte cellulaire transformé et lesdits ligands déterminés.

Le procédé décrit ci-dessus permet également l'identification des "fragments contenant les sites" dont question précédemment et qui ne comportent qu'une partie de la séquence de 402 acides aminés de la figure 1.

Cette identification consiste a utiliser des insérats de taille plus réduite que l'acide nucléique codant pour la susdite séquence, à mettre en oeuvre les étapes relatives à l'expression, au transport du produit d'expression et à l'exposition rappelées ci-dessus. Lorsqu'on obtient l'expression, le transport du produit d'expression et son exposition sur la membrane, ainsi que la réaction avec les ligands telle que précédemment définie, on peut déterminer ainsi les fragments contenant les sites essentiels. Par conséquent, l'absence de réaction avec les ligands, telle que précédemment définie, en utilisant des fragments de taille plus réduite que la séquence complète, tendrait à montrer qu'on a éliminé certains sites essentiels.

L'invention concerne également un kit pour la détection de l'affinité éventuelle d'un ligand pour un polypeptide de l'invention , lequel kit comprend:
- une culture d'hôtes cellulaires transformés par un vecteur modifié tel que défini précédemment ou une culture d'hôtes cellulaires définis précédemment,
- des moyens physiques ou chimiques pour induire l'expression de la séquence nucléotidiques contenue dans le vecteur modifié lorsque le promoteur placé en amont de cette séquence est un promoteur inductible par lesdits moyens physiques ou chimiques, et obtenir une protéine,
- un ou plusieurs ligands témoins ayant des affinités déterminées pour le susdit polypeptide,
- des moyens physiques ou chimiques pour la caractérisation de l'activité biologique de la protéine exprimée.

L'invention concerne également un procédé de criblage de médicaments destinés au traitement de l'obésité, du diabète gras, ainsi que des hyperlipidémies.

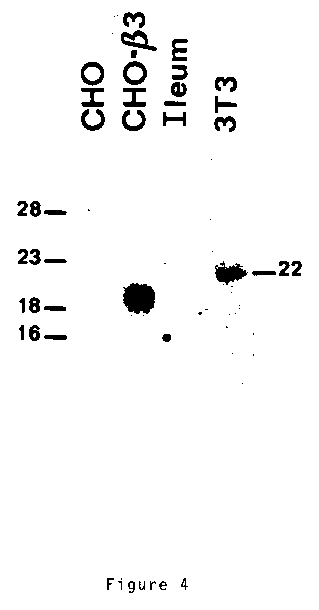

D'autres caractéristiques et avantages de l'invention apparaissent dans la suite de la description et des exemples, notamment en rapport avec les dessins et tableaux dans lesquels :
- La Figure 1 représente les séquences d'acides aminés du récepteur adrénergique β1 (deuxième ligne) humain, du récepteur adrénergique β2 humain (troisième ligne), alignées avec celles du récepteur adrénergique β3 humain (première ligne). Dans les séquences des récepteurs adrénergiques β1 et β2, les résidus d'amino acides identiques et en position analogue à ceux du récepteur adrénergique β3 sont marqués par des traits pointillés.
   Pour mettre en évidence les homologies, on a effectué des délétions dans les trois séquences, et ces délétions sont représentées par les espaces entre crochets.
   Les sept régions hydrophobes de 21 à 27 résidus qui sont considérées comme formant des domaines transmembranaires en hélice a sont indiquées avec des traits pleins gras et délimitent des boucles extra-cellulaires et intra-cellulaires, les boucles extra-cellulaires étant représentées par 01-04 sur la Figure et les boucles intra-cellulaires par i1 à i4.
   Dans les régions N-terminale extra-cellulaires des trois récepteurs, les séquences consensus pour les sites de glycosylation liés à l'asparagine (NXS/T) sont soulignées, étant rappelé que dans la représentation des acides aminés selon DAYHOFF, N représente l'asparagine, S représente la sérine, T représente la thréonine, X représente n'importe quel acide aminé.
   Dans la troisième boucle intra-cellulaire et dans la région C-terminale, des triangles montrent les résidus Ser et Thr qui sont problablement impliqués dans la désensibilisation du récepteur.
- La Figure 2 représente l'accumulation d'AMP cyclique dans des cellules CHO-β3 après exposition à A, (1)-isoproterenol (▲), (1) -noradrénaline (○), (1) -adrénaline (·) ou, (d)-isoproterenol (v) et B;BRL 7344 (■), oxprenolol (□), pindolol (○) ou salbutamol (·). Les valeurs K_{act}(nM) sont : 3.86 ± 0.43 pour le (1)-isoproterenol, 5.65 ± 1.32 pour le BRL 37344, 6.28 ± 0.80 pour la noradrénaline, 47.4 ± 6.9 pour l'adrénaline, 76.3 ± 1.57 pour l'oxprenolol, 108 ± 3 pour le (d)-isoproterenol, 167 ± 10 pour le pindolol et 273 ± 25 pour le salbutamol.
   Dans ces expériences, l'accumulation d'AMP cyclique varie de 300% à 400% par rapport à des niveaux de base et les résultats sont exprimés en % de réponse maximale (moyenne ± écart type [n=4 à 6]) induite par chacun des ligands. Dans les cellules CHO, ni la noradrénaline, ni l'adrénaline, ni l'isoproterenol n'activent l'adénylate cyclase.
   L'agoniste procaterol spécifique de β2 stimule l'adénylate cyclase à des concentrations supérieures à 10⁻⁵M (non représenté).
   C; stimulation de cyclase par isoproterenol, BRL 37344, BRL 28410 et salbutamol dans des cellules CHO exprimant des gènes β1, β2 ou β3. Pour chacun des panneaux, les résultats sont la moyenne de deux à trois expériences faites deux fois. La moitié de l'écart type est représentée pour chacun par des barres verticales. Trois concentrations de ligands ont été utilisées : 5x10⁻⁹(1), 5x10⁻⁷ (2) et 10⁻⁴ (3).
   Les résultats représentent le % [moyenne ± écart type (n=4 à 6)] de la réponse induite par l'isoproterenol 10⁻⁴M.
   La méthode utilisée est la suivante : la région codante du gène du récepteur adrénergique β3 a été insérée sous contrôle d'un promoteur SV40 dans un vecteur d'expression (20) qui contient également le gène de dihydrofolate réductase murine (DHFR) utilisé pour la sélection des cellules transfectantes. Cette construction a été introduite dans des cellules de CHO (21) déficientes en DHFR, donnant des colonies résistantes au milieu sélectif à partir duquel des sous-clones stables sont obtenus. L'expression gène récepteur adrénergique β3 est démontrée par hybridation de l'ARN à une sonde spécifique du récepteur adrénergique β3 (Figure 4). Pour le test de l'AMP cyclique, des cellules sont cultivées et récoltées après traitement avec Versene/EDTA (Eurobio, Paris), lavées et resuspendues dans un milieu de Hank contenant 20 mM d'Hepes tamponnés à pH 7.4, de l'acide ascorbique 1 mM et de l'isobutyl-méthyle-xanthine 0.1mM. Des aliquotes de 10⁶ cellules sont incubées pendant 30 min., dans un volume total de 1 ml avec des concentrations indiquées d'agonistes et des niveaux de cAMP sont mesurés selon les spécifications de la trousse d'essai Amersham.

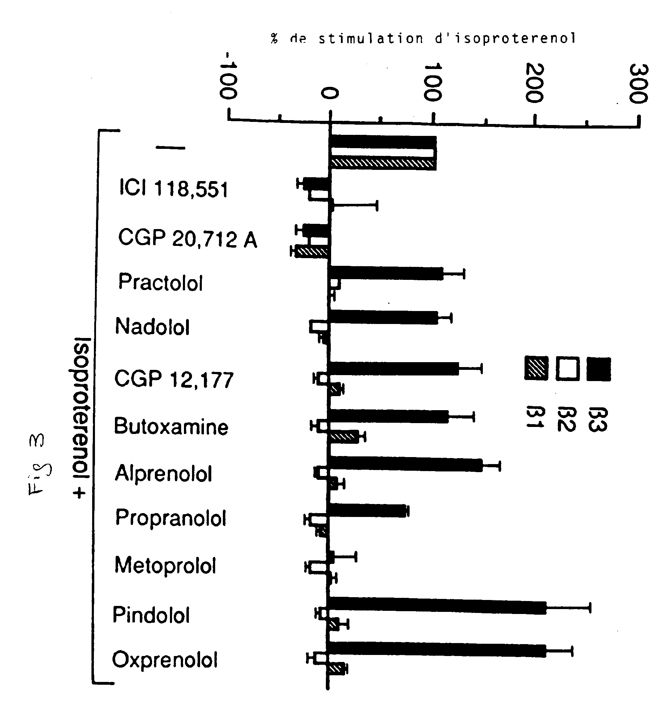

- La Figure 3 représente l'inhibition par les antagonistes β-adrénergiques de l'accumulation d'AMP cyclique induite par l'isoproterenol dans des cellules de CH0-β1, CHO-β2 et CHO-β3. Les résultats sont exprimés en % (moyenne ± SD (n=4 à 6)] d'accumulation d'AMP cyclique induite par l'isoproterenol seul. Les composés ICI, 118551 (fabriqué par la société IMPERIAL CHEMICAL INDUSTRY) et CGP 20712A (fabriqué par la société CIBA-GEIGY), bloquent l'accumulation d'AMP cyclique avec des Ki respectif calculés de 0,77 ± 0,08µM et de 6,70 ± 0,87 µM. Le metoprol n'a pas d'effet à des concentrations inférieures à 10⁻⁵M.
   La méthode utilisée est la suivante : les cellules, comme dans la Figure 2, sont incubées pendant 30 min., avec les inhibiteurs à 10⁻⁴M avant l'addition de 5.10⁻⁹ M d'isoproterenol. L'incubation est poursuivie encore pendant 30 min., et on mesure les niveaux d'AMP cyclique.
- La Figure 4 représente des analyses de Northern Blot d'ARN de tissus et de lignées cellulaires. L'ARN à analyser provient de tissus de rats (2µg d'ARN polyA+ de : cerveau total, cortex, hippocampe, hypothalamus, hypophyse, bulbe olfactif, striatum, cervelet, iléon, foie, coeur, poumon, rein, peau et muscle) et de lignées cellulaires humaines (5µg d'ARN polyA+ de : neuroblastomes, poumon et fibroblastes de poumon et d'épiderme, lymphocytes B et T, erythroblastes et myeloblastes). Aucun de ces ARN ne donne de signal détectable (les résultats sont montrés pour l'ileum). Un message spécifique est détecté dans l'ARN (15µG d'ARN total) à partir de lignées de cellules adipeuses 3T3-F442A ou à partir de cellules de CHO-β3 transfectées. Les valeurs S pour les marqueurs de poids moléculaire d'ARN sont représentés dans la marge gauche.

La méthode utilisée est la suivante : l'ARN est soumis à l'électrophorèse sur gel d'agarose à 0.7%, transféré sur des membranes de nylon et hybridé avec des sondes spécifiques des récepteurs adrénergiques β3 marqués radioactivement par la méthode d'initiation aléatoire. Cette sonde dérive de la troisième boucle intracytoplasmique du récepteur adrénergique β3 dont la séquence est faiblement conservée entre les trois récepteurs (positions 1313 à 1513 de la Figure 1bis). Suite à l'hybridation, les filtres sont lavés dans 0.1xSSC (c'est-à-dire 0.015 M NaCl et 0.0015 M citrate de sodium, pH 7.0) +0.05% SDS à 55°C. Dans ces conditions, aucun signal n'est obtenu avec l'ARN préparé à partir de cellules de CHO exprimant le récepteur adrénergique β1 ou β2.

### EXEMPLE :

Détermination et expression du récepteur adrénergique β3 humain dans des cellules CHO :

On a investigué une banque génomique humaine avec les régions entières codantes du gène du récepteur adrénergique β1 de la dinde (14) et du gène humain du récepteur adrénergique β2 (8). Les gènes codant respectivement pour les récepteurs adrénergiques humains β1 et β2 ont été identifiés parmi les clones positifs. L'homologie entre leurs régions codantes est de 48.9%. On a obtenu d'autres clones contenant un gène sans intron, dont la région codante présente respectivement 50.7% et 45.5% d'homologie avec les régions codantes de β1 et β2 (Figure 1). Ce gène a été désigné par gène du récepteur adrénergique β3. Plus précisément, sur la Figure 1, chaque groupe de trois lignes correspond pour la première ligne à β3, pour la seconde ligne à β1 et pour la troisième ligne à β2.

Le gène du récepteur adrénergique β3 code pour un polypeptide de 402 résidus amino acides (poids moléculaire 42881D) qui présente les principales caractéristiques communes aux autres récepteurs membranaires de la famille R₇ G (Figure 1). 11 possède sept groupes, de 21 à 27 amino acides essentiellement hydrophobes, susceptibles de constituer des domaines transmembranaire en hélice α. Ces domaines transmembranaires jouent un rôle important dans la formation des sites de reconnaissance des catécholamines par les récepteurs β-adrénergiques (15-18) et sont assurément les régions les plus homologues entre les trois protéines β1, β2 et β3 (Figure 1). En particulier, les résidus Asp en position 79 et 113 du récepteur adrénergique β2, qui peuvent agir comme des contre-ions vis-à-vis de l'amine chargée positivement des ligands adrénergiques (18), sont conservés aux positions analogues du récepteur adrénergique β3, D'autres résidus fonctionnellement importants tels que les résidus Cys en position 106, et 184, Asn en position 318 et Pro en position 323 de la séquence du récepteur adrénergique β2 sont présents aux positions correspondantes sur le récepteur adrénergique β3.

Comme les autres protéines R₇G, le récepteur adrénergique β3 comprend dans sa région amino terminale des séquences consensus pour les sites de glycosylation liés à l'Asn. Dans sa troisième boucle intra-cytoplasmique et dans sa région C-terminale, le récepteur adrénergique β3 présente plusieurs résidus Ser et Thr, encadrés par des résidus basiques (Arg et Lys) et des résidus cassant les structures en hélice α (Pro et Gly), qui peuvent servir de substrats pour les kinases éventuellement engagées dans la désensibilisation du récepteur (19).

Pour mieux caractériser le récepteur adrénergique β3, son gène a été transfecté dans des cellules CHO et plusieurs clones, produisant l'ARN correspondant, ont été stabilisés. Ces cellules sont désignées par cellules "CHO-β3". Des cellules CHO exprimant soit le gène du récepteur adrénergique β1, soit le gène du récepteur adrénergique β2, ont été également préparées pour comparer les propriétés pharmacologiques des trois récepteurs dans un environnement identique. Ces cellules sont désignées par cellules CHO-β1 et CHO-β2. Les cellules CHO-β3 synthétisent une protéine de poids moléculaire apparant d'environ 65000D, qui peut être visualisée par marquage d'affinité avec l'(¹²⁵I)-iodocyanopindolol-diazirine. L'addition post-transcriptionnelle de résidus glucidiques à l'un ou aux deux sites de glycosylation de l'extrémité amino-terminale du récepteur adrénergique β3, est certainement responsable de ce poids moléculaire supérieur à celui déduit de la séquence d'acides aminés.

L'exposition des cellules CHO-β3 aux agonistes β-adrénergiques (Figure 2) tels que l'adrénaline, la noradrénaline, l'isoproterenol, le salbutamol, le BRL 28410 ou le BRL 37344 accroît la concentration intracellulaire d'AMP cyclique de 300% à 400% par rapport à des niveaux de base, tandis qu'il n'y a pas d'influence sur des cellules CHO non transfectées. Cet effet est stéréospécifique, étant donné que l'isoproterenol-(l) est presque 30 fois plus puissant que le (d)-isoproterenol vis-à-vis de l'activation de la cyclase. D'autres agonistes-β tels que le procaterol et le CGP 361A conduisent à une stimulation de cyclase seulement lorsqu'ils sont utilisés à des concentrations supérieures à 10⁻⁵M.

L'ordre dans lequel les composés suivants : (l)-isoproterenol, BRL 37344, BRL 28410 et salbutamol activent l'adénylate cyclase dans les cellules CHO-β3 est clairement différent de celui obtenu pour les réponses médiatisées par les récepteurs adrénergiques β1 ou β2 (Figure 2C). Pour les cellules CHO-β3, cet ordre est voisin de celui déterminé pour la stimulation de la lipolyse des cellules adipeuses de rats. La seule différence est l'ordre relatif de l'isoproterenol comparé au BRL 37344. Cependant, dans la référence citée, c'est le (dl)-isoproterenol racémique qui a été utilisé alors que, dans l'invention, on a utilisé le lévogyre (l)-isoproterenol.

On a effectué l'analyse dans des cellules CHO-β3, de la capacité de plusieurs β-antagonistes classiques à bloquer l'activation de l'adénylate cyclase stimulée par l'isoproterenol (Figure 3). A part l'ICI 118,551, le CGP 20,712A et le metoprolol, aucun de ces composés utilisés à 10⁻⁴M n'est capable d'inhiber cet effet. A des concentrations inférieures à 10⁻⁵M, le metoprolol n'a plus d'effets et l'ICI 118,551 et le CGP 20,712A bloquent l'accumulation d'AMP cyclique avec des Ki respectifs de 0.77 ± 0.08 µM et 6.70 + 0.87 µM. En accord avec ces résultats, ni les cellules CHO-β3 intactes, ni leurs fractions membranaires ne représentent de liaison spécifique et saturable vis-à-vis de l'alprenolol ou du CGP 12,177 marqué au ³H. De plus, le K_{D} de I¹²⁵iodocyanopindolol pour le récepteur adrénergique β3 (488 ± 90 pM) est environ 10 fois supérieur à celui pour les récepteurs adrénergiques β1 ou β2.

Selon des études in vitro, le pindolol et l'oxprenolol sont considérés comme des antagonistes β-adrénergiques. Cependant, ils sont aussi considérés comme des agonistes partiels puisqu'in vivo ils peuvent présenter une légère activité sympathomimétique (22). Dans les cellules CHO transfectées avec un seul type de récepteur, ces composés sont complètement agonistes vis-à-vis du récepteur adrénergique β3 (Figure 2), tandis qu'ils bloquent totalement l'accumulation d'AMP cyclique médiatisée par les récepteurs adrénergiques β1 et β2 (Figure 3).

Ce nouveau sous-type de récepteur adrénergique β, pourrait moduler différentes fonctions telles que la lipolyse, la sécrétion d'insuline ou la relaxation intestinale. Les contractions de l'iléon de cobaye induites par la voie cholinergique sont modulées par les agonistes adrénergiques. Cependant, en dépit du blocage total avec la phentolamine et le propanolol des récepteurs α et β adrénergiques, l'isoproterenol peut toujours inhiber la survenue des contractions. Le récepteur β3 présente une affinité faible pour le propanolol et d'autres β bloquants classiques, mais une réponse marquée vis-à-vis de l'agoniste BRL 37344, qui est un puissant stimulant de la lipolyse dans le tissu adipeux (1).

L'hypothèse selon laquelle le récepteur β3 est présent dans les tissus adipeux a été confirmée par l'analyse de la capacité de l'ARN provenant de tissus d'origines diverses à s'hybrider à des sondes spécifiques de récepteur adrénergique β3 (Figure 4): un signal d'hybridation avec les sondes utilisées a été observé uniquement avec l'ARN de la lignée adipocytaire 3T3-F442A (23).

Ce récepteur adrénergique β3 pourrait également être impliqué dans la régulation par les catécholamines de l'action de l'insuline sur le métabolisme du glucose et des acides gras.

### REFERENCES

1. Arch, J.R.S., Ainsworth, A.T., Cawthorne, M.A., Piercy, V., Sennitt, M.V., Thody, V.E., Wilson, C. and Wilson, S. (1984), *Nature* **309**, 163-165.
2. Jacobson, B., Vauquelin, G., Wesslau, C., Smith, U. and Strosberg, A.D. (1981), *Eur. J. Biochem.* **114**, 349-354.
3. Bond, R.A. and Clarke, D.E. (1987), *Br. J. Pharmac.* **91**, 683-686.
5. Cotecchia, S., Schwinn, D.A., Randall, R.R., Lefkowitz, R.J., Caron, M.G. and Kobilka, B.K. (1988), *Proc. Natl. Acad. Sci. USA* **85**, 7159-7163
6. Kobilka, B.K., Matsui, H., Kobilka, T.L, Yang.Feng, T.L, Francke, U., Caron, M.G., Lefkowitz, R.J. and Regan, J.W. (1987), *Science* **238**, 650-656.
7. Frielle, T., Collins, S., Daniel, K.W., Caron, M.G., Lefkowitz, R.J. and Kobilka, B.K. (1987), *Proc. Natl. Acad. Sci. USA* **84**, 7920-7924.
8. Emorine, L.J., Marullo, S., Delavier-Klutchko, C., Kaveri, S.V., Durieu-Trautman, O. and Strosberg, A.D. (1987), *Proc. Natl. Acad. Sci. USA* **84**, 6995-6999.
9. Dixon, R.A.F., Strader, C.D. and Sigal, I.S. (1988), *Annual Reports in Medicinal chemistry,* 221-233. Ed. Seamon, K.B., Food and Drug Administration, Bethesda, Md 20892.
10. Emorine, L.J., Marullo, S., Sutren, M., Delavier, C., Eshdat, Y., Raposo, G. and Strosberg, A.D. (1988), Proc. NATO Adv. Res. Workshop: "Molecular biology of neuroreceptors and ion channels", Santorini, Ed. by A., Maelicke (in the press).
11. Bonner, T.I., Young, A.C., Brann, M.R. and Buckley, N.J. (1988), *Neuron* **1**, 403-410.
12. Regan, J.W., Kobilka, T.S., Yang-Feng, T.L., Caron, M.G., Lefkowitz, R.J. and Kobilka, B.K. (1988), *Proc. Natl. Acad. Sci. USA* **85**, 6301-6305.
13. Bahouth, S.W. and Malbon, C.C. (1988), *Molec. Pharmacol.* **34**, 318-328.
14. Yarden, Y., Rodriguez, H., Wong, S.K.F., Brandt, D.R., May, D.C., Burnier, J., Harkins, R.N., Chen, E.Y., Ramachandran, J., Ullrich, A. and Ross, E.M. (1986), *Proc. Natl. Acad. Sci. USA* **83**, 6795-6799.
15. Dixon, R.A.F., Sigal, I., Candelore, M.R., Register, R.B., Scattergood, W., Rands, E. and Strader, C.D. (1987), *EMBO J.* **6**, 3269-3275.
16. Dohlman, H.G., Caron, M.G., Strader, C.D., Amlaiky, N. and Lefkowitz, R.J. (1988), *Biochem.* **27**, 1813-1817.
17. Kobilka, B.K., Kobilka, T.S., Daniel, K., Regan, J.W., Caron, M.G. and Lefkowitz, R.J. (1988), *Science* **240**, 1310-1316.
18. Strader,C.D., Sigal, I.S., Candelore, M.R., Rands, E., Hill, W.S. and Dixon, R.A.F. (1988), *J. Biol. Chem.* **263** 10267-10271.
19. Bouvier, M., Hausdorff, P., De Blasi, A., O'Dowd, B.F., Kobilka, B.K., Caron, M.G. and Lefkowitz, R.J. (1988), *Nature* **333**, 370-373.
20. Larsky, LA., Dowbenko, D., Simonsen, C.C. and Berman, P.W. (1984), *Biotechnology* **2**, 527-532.
21. Urlaub, G. and Chasin, LA. (1980), *Proc. Natl. Acad. Sci. U.S.A.* **77**, 4216-4220.
22. Goodman and Gilman's (1980), *The pharmacological basis of therapeutics* 6^{th} ed., Macmillan Publishing Co., Inc.
23. Green,H. and Kehinde, O. (1976), *Cell* **7**, 105-113.

## Revendications

1. Polypeptide ayant une activité de récepteur β-adrénergique contenant :
. la séquence de 402 acides aminés de la Figure 1,
. ou un fragment de cette séquence, ce fragment étant tel que
* soit il contient néanmoins les sites contenus dans cette séquence et dont la présence est nécessaire pour que, lorsque ce fragment est exposé à la surface d'une cellule, il soit capable de participer à l'activation de l'adénylate cyclase en présence d'un agoniste, cette activation étant croissante dans l'ordre des agonistes suivants : salbutamol, BRL 28410, BRL 37344 et (1)-isoproterenol,
* soit il est susceptible d'être reconnu par des anticorps qui reconnaissent également la susdite séquence de 402 acides aminés, mais ne reconnaissent ni le récepteur adrénergique β1, ni le récepteur adrénergique β2.
* soit il est susceptible de générer des anticorps reconnaissant la susdite séquence de 402 acides aminés mais ne reconnaissant ni le récepteur adrénergique β1, ni le récepteur adrénergique β2.

2. Polypeptide selon la revendication 1, dont la capacité à stimuler l'adénylate cyclase et/ou l'accumulation de cAMP induite par l'isoproterenol, n'est pas inhibée par les composés suivants:
- practolol,
- nadolol,
- CGP 12,177,
- butoxamine,
- alprenolol,
- propanolol,
- pindolol,
- oxprenolol,
utilisés à des concentrations inférieures ou égales à 10⁻⁴M, dans les conditions suivantes :
en ce qui concerne l'accumulation de l'AMP cyclique, des cellules sont cultivées et récoltées après traitement avec Versene/EDTA (Eurobio, Paris), lavées et resuspendues dans un milieu de Hank contenant 20 mM d'Hepes tamponnés à pH 7.4, de l'acide ascorbique 1 mM et de l'isobutyl-méthyle-xanthine 0.1 mM; des aliquotes de 10⁶ cellules sont incubées pendant 30 minutes, avec les inhibiteurs à 10⁻⁴M avant l'addition de 5.10⁻⁹M d'isoproterenol ; l'incubation est poursuivie encore pendant 30 minutes, et on mesure les niveaux d'AMP cyclique par exemple selon les spécifications de la trousse d'essais Amersham.

3. Polypeptide selon la revendication 1, caractérisé en ce que le pindolol et l'oxprenolol stimulent l'adénylate cyclase et/ou l'accumulation d'AMP cyclique dans des cellules CHO transfectées avec le susdit polypeptide, dans les conditions suivantes :
en ce qui concerne l'accumulation de l'AMP cyclique, des cellules sont cultivées et récoltées après traitement avec Versene/EDTA (Eurobio, Paris), lavées et resuspendues dans un milieu de Hank contenant 20 mM d'Hepes tamponnés à pH 7.4, de l'acide ascorbique 1 mM et de l'isobutyl-méthyle-xanthine 0.1 mM des aliquotes de 10⁶ cellules sont incubées pendant 30 minutes, dans un volume total de 1 ml avec des concentrations de pindolol ou d'oxprenolol supérieures à 10⁻¹¹ M et les niveaux de cAMP sont mesurés par exemple selon les spécifications de la trousse d'essai Amersham.

4. Polypeptide selon la revendication 1, caractérisé en ce qu'il est constitué par la séquence, représentée sur la Figure 1, s'étendant de l'extrémité constituée par l'acide aminé en position 1 à celle constituée par l'extrémité en position 402.

5. Acide nucléique caractérisé en ce qu'il comprend ou est constitué par un enchaînement de nucléotides codant pour les polypeptides selon les revendications 1 à 4.

6. Acide nucléique selon la revendication 5, caractérisé en ce qu'il comprend ou est constitué par l'enchaînement de nucléotides représenté sur la Figure 1 bis, s'étendant de l'extrémité constituée par le nucléotide 1 à celle constituée par le nucléotide 2022.

7. Vecteur recombinant, en particulier pour le clonage et/ou l'expression, notamment du type plasmide, cosmide ou phage, caractérisé en ce qu'il contient un acide nucléique selon l'une des revendications 5 et 6 en l'un de ses sites non essentiels pour sa réplication.

8. Vecteur recombinant selon la revendication 7, caractérisé en ce qu'il s'agit du vecteur M13mp18-Huβ3, (n°1085), constitué par un bactériophage déposé sous le n°I-833 le 20 janvier 1989 à la CNCM, 25 rue du Docteur Roux, Paris.

9. Vecteur recombinant selon la revendication 7, caractérisé en ce qu'il contient en l'un de ses sites non essentiels pour sa réplication des éléments nécessaires pour promouvoir l'expression d'une séquences d'acides aminés selon les revendications 1 à 4, dans un hôte cellulaire et éventuellement un promoteur reconnu par les polymérases de l'hôte cellulaire, en particulier un promoteur inductible et éventuellement une séquence signal et une séquence d'ancrage.

10. Hôte cellulaire transformé par un vecteur recombinant selon l'une quelconque des revendications 7 à 9 et comprenant les éléments de régulation permettant l'expression de la séquence nucléotidique codant pour le polypeptide selon l'une des revendications 1 à 4 dans cet hôte.

11. Hôte cellulaire transformé selon la revendication 10, caractérisé en ce qu'il est choisi parmi les bactéries, notamment E. coli.

12. Hôte cellulaire transformé selon la revendication 10, caractérisé en ce qu'il est choisi parmi les organismes eucaryotes tels que des cellules CHO.

13. Anticorps caractérisé(s) en ce qu'il(s) est (ou sont) dirigé(s) de façon spécifique contre un polypeptide selon l'une quelconque des revendications 1 à 4, et en ce qu'il(s) ne reconnait (ou reconnaissent) ni le récepteur adrénergique β1, ni le récepteur adrénergique β2 et en particulier celui ou ceux reconnaissant les séquences d'acides aminés suivantes:
1 à 36 178 à 201
64 à 74 223 à 291
101 à 108 314 à 325
133 à 135 345 à 402

14. Sonde nucléotidique caractérisée en ce qu'elle s'hybride avec l'un des acides nucléiques selon les revendications 5 et 6 ou leur séquence complémentaire dans les conditions d'hybridation telles qu'elle ne s'hybride pas aves les gènes ou ARN messager des récepteurs β1 ou β2 adrénergiques, et en particulier les sondes choisies parmi les sondes nucléotidiques suivantes :
. - celle définie par la séquence d'acides nucléiques représentée sur la Figure 1bis, s'étendant de l'extrémité constituée par le nucléotide en position 1, à celle constituée par le nucléotide en position 637
. - celle définie par la séquence d'acides nucléiques représentée sur la Figure 1bis, s'étendant de l'extrémité constituée par le nucléotide en position 638 à celle constituée par le nucléotide en position 745,
. - celle définie par la séquence d'acides nucléiques représentée sur la Figure 1bis, s'étendant de l'extrémité constituée par le nucléotide en position 1313, à celle constituée par le nucléotide en position 1513,
. - celle définie par la séquence d'acides nucléiques représentée sur la Figure 1bis, s'étendant de l'extrémité constituée par le nucléotide en position 1679, à celle constituée par le nucléotide en position 1843,
. - celle définie par la séquence d'acides nucléiques représentée sur la Figure 1bis, s'étendant de l'extrémité constituée par le nucléotide en position 1844, à celle constituée par le nucléotide en position 2022,
. - celle définie par la séquence d'acides nucléiques représentée sur la Figure 1bis, s'étendant de l'extrémité constituée par le nucléotide en position 638, à celle constituée par le nucléotide en position 1843.
ou leur séquence nucléotidique complémentaire.

15. Procédé de préparation d'un polypeptide selon quelconque des revendications 1 à 4, caractérisé en ce que l'on cultive dans un milieu approprié un hôte cellulaire préalablement transformé par un vecteur recombinant contenant un acide nucléique selon l'une quelconque des revendications 7 à 9,
et on récupère à partir de la susdite culture le polypeptide produit par ledit hôte cellulaire transformé.

16. Procédé de détection de la capacité d'une molécule à se comporter comme ligand vis-à-vis d'un polypeptide selon l'une quelconque des revendications 1 à 4, caractérisé par :
- la mise en contact de la molécule avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avèrerait effectivement posséder une affinité pour ce polypeptide,
- la détection de la formation éventuelle d'un complexe du type ligand-polypeptide.

17. Procédé pour l'étude de l'affinité d'un polypeptide selon l'une quelconque des revendications 1 à 4, pour un plusieurs ligands déterminés, caractérisé par :
- la transformation d'un hôte cellulaire compétent avec un vecteur, notamment un plasmide ou un phage, dans lequel avait auparavant été insérée une séquence de nucléotides ou codant pour le récepteur β3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur β3 exprimé vers la membrane de façon telle que les séquences transmembranaires de récepteur β3 soient exposées à la surface de l'hôte cellulaire transformé,
- la mise en contact de cet hôte cellulaire avec ces ligands déterminés,
- la détection d'une réaction affine entre ledit hôte cellulaire transformé et lesdits ligands déterminés.

18. Kit pour la détection de l'affinité éventuelle d'un ligand pour un polypeptide selon l'une quelconque des revendications 1 à 4, comprenant :
- une culture d'hôtes cellulaires transformés par un vecteur modifié tel que défini dans l'une quelconque des revendications 10 à 12 ou une culture d'hôtes cellulaires selon la revendication 12,
- des moyens physiques ou chimiques pour induire l'expression de la séquence nucléotidiques contenue dans le vecteur modifié lorsque le promoteur placé en amont de cette séquence est un promoteur inductible par lesdits moyens physiques ou chimiques, et obtenir une protéine,
- un ou plusieurs ligands témoins ayant des affinités déterminées pour le susdit polypeptide,
- des moyens physiques ou chimiques pour la caractérisation de l'activité biologique de la protéine exprimée.

## Claims

1. Polypeptide having a β-adrenergic receptor activity containing:
. the sequence of 402 amino acids of Figure 1,
. or a fragment of this sequence, this fragment being such that
* either it contains nevertheless the sites contained in this sequence and whose presence is necessary for it to be capable, when this fragment is exposed at the surface of a cell, of participating in the activation of adenylate cyclase in the presence of an agonist, this activation increasing in the order of the following agonists; salbutamol, BRL 28410, BRL 37344 and (1)-isoproterenol,
* or it is capable of being recognized by antibodies which also recognize the abovementioned sequence of 402 amino acids, but recognize neither the β1-adrenergic receptor nor the β2-adrenergic receptor,
* or it is capable of generating antibodies which recognize the abovementioned sequence of 402 amino acids but recognizes neither the β1-adrenergic receptor nor the β2-adrenergic receptor.

2. Polypeptide according to Claim 1, whose capacity to stimulate adenylate cyclase and/or the accumulation of cAMP induced by isoproterenol is not inhibited by the following compounds:
- practolol,
- nadolol,
- CGP 12.177,
- butoxamine,
- alprenolol,
- propanolol,
- pindolol,
- oxprenolol,
used at concentrations of less than or equal to 10⁻⁴M, under the following conditions:
with regard to the accumulation of cyclic AMP, cells are cultured and harvested after treatment with Versene/EDTA (Eurobio, Paris), washed and resuspended in a Hank's medium containing 20 mM of Hepes buffered to pH 7.4, 1 mM of ascorbic acid and 0.1 mM of isobutylmethyl-xanthine; aliquots of 10⁶ cells are incubated for 30 minutes with the inhibitors at 10⁻⁴M before the addition of 5x10⁻⁹M of isoproterenol; the incubation is continued for a further 30 minutes and the cyclic AMP levels are measured for example according to the specifications of the Amersham test kit.

3. Polypeptide according to Claim 1, characterized in that pindolol and oxprenolol stimulate adenylate cyclase and/or the accumulation of cyclic AMP in CHO cells transfected with the abovementioned polypeptide, under the following conditions:
with regard to the accumulation of cyclic AMP, cells are cultured and harvested after treatment with Versene/EDTA (Eurobio, Paris), washed and resuspended in a Hank's medium containing 20 mM of Hepes buffered to pH 7.4, 1 mM of ascorbic acid and 0.1 mM of isobutylmethyl-xanthine; aliquots of 10⁶ cells are incubated for 30 minutes in a total volume of 1 ml with pindolol or oxprenolol concentrations greater than 10⁻¹¹M and the cAMP levels are measured for example according to the specifications of the Amersham test kit.

4. Polypeptide according to Claim 1, characterized in that it consists of the sequence, represented in Figure 1, extending from the end consisting of the amino acid in position 1 to that consisting of the end in position 402.

5. Nucleic acid characterized in that it comprises or consists of a chain of nucleotides encoding the polypeptides according to Claims 1 to 4.

6. Nucleic acid according to Claim 5, characterized in that it comprises or consists of the chain of nucleotides represented in Figure 1bis, extending from the end consisting of nucleotide 1 to that consisting of nucleotide 2022.

7. Recombinant vector, in particular for cloning and/or expression, especially of the plasmid, cosmid or phage type, characterized in that it contains a nucleic acid according to one of Claims 5 and 6 at one of its sites which are not essential for its replication.

8. Recombinant vector according to Claim 7, characterized in that it is the vector M13mp18-Huβ3 (No. 1085), consisting of a bacteriophage deposited under No. I-833 on 20 January 1989 at CNCM, 25 rue du Docteur Roux, Paris.

9. Recombinant vector according to Claim 7, characterized in that it contains at one of its sites which are not essential for its replication, elements necessary for promoting the expression of an amino acid sequence according to Claims 1 to 4, in a cellular host and optionally a promoter recognized by the polymerases of the cellular host, in particular an inducible promoter and optionally a signal sequence and an anchoring sequence.

10. Cellular host transformed by a recombinant vector according to any one of Claims 7 to 9 and comprising the regulatory elements which permit the expression of the nucleotide sequence encoding the polypeptide according to one of Claims 1 to 4 in this host.

11. Cellular host transformed according to Claim 10, characterized in that it is. chosen from bacteria, especially E. coli.

12. Transformed cellular host according to Claim 10, characterized in that it is chosen from eukaryotic organisms such as CHO cells.

13. Antibody (antibodies) characterized in that it is (they are) directed specifically against a polypeptide according to any one of Claims 1 to 4, and in that it (they) recognizes (recognize) neither the β1-adrenergic receptor nor the β2-adrenergic receptor and in particular that or those which recognize the following amino acid sequences:
1 to 36 178 to 201
64 to 74 223 to 291
101 to 108 314 to 325
133 to 135 345 to 402

14. Nucleotide probe characterized in that it hybridizes with one of the nucleic acids according to Claims 5 and 6 or their complementary sequence under hybridization conditions such that it does not hybridize with the genes or messenger RNA for the β1- or β2-adrenergic receptors, and in particular the probes chosen from the following nucleotide probes:
- that defined by the nucleic acid sequence represented in Figure 1bis, extending from the end consisting of the nucleotide in position 1, to that consisting of the nucleotide in position 637,
- that defined by the nucleic acid sequence represented in Figure 1bis, extending from the end consisting of the nucleotide in position 638, to that consisting of the nucleotide in position 745,
- that defined by the nucleic acid sequence represented in Figure 1bis, extending from the end consisting of the nucleotide in position 1313, to that consisting of the nucleotide in position 1513,
- that defined by the nucleic acid sequence represented in Figure 1bis, extending from the end consisting of the nucleotide in position 1679, to that consisting of the nucleotide in position 1843,
- that defined by the nucleic acid sequence represented in Figure 1bis, extending from the end consisting of the nucleotide in position 1844, to that consisting of the nucleotide in position 2022,
- that defined by the nucleic acid sequence represented in Figure 1bis, extending from the end consisting of the nucleotide in position 638, to that consisting of the nucleotide in position 1843,
or their complementary nucleotide sequence.

15. Process for preparing a polypeptide according to any one of Claims 1 to 4, characterized in that a cellular host previously transformed by a recombinant vector containing a nucleic acid according to any one of Claims 7 to 9 is cultured in an appropriate medium, and the polypeptide produced by the said transformed cellular host is recovered from the abovementioned culture.

16. Process for detecting the capacity of a molecule to behave as ligand towards a polypeptide according to any one of Claims 1 to 4, characterized by:
- the placing of the molecule in contact with a cellular host previously transformed by a vector itself modified by an insert encoding the abovementioned polypeptide, this host carrying at its surface one or more sites specific for this polypeptide, where appropriate after inducing the expression of this insert, this placing in contact being carried out under conditions which permit the formation of a bond between at least one of these specific sites and the said molecule so long as it was effectively shown to possess an affinity for this polypeptide,
- the detection of the possible formation of a ligand-polypeptide type complex.

17. Process for studying the affinity of a polypeptide according to any one of Claims 1 to 4, for one or more determined ligands, characterized by:
- the transformation of a competent cellular host with a vector, especially a plasmid or a phage, into which there had previously been inserted a nucleotide sequence encoding the β3-receptor (insert), under the control of regulatory elements, especially a promoter recognized by the polymerases of the cellular host and which permit the expression of the said nucleotide sequence in the cellular host used,
- the culture of the cellular host transformed under conditions which permit the expression of the said insert, and the transport of the expressed β3-receptor towards the membrane so that the transmembrane sequences of the β3-receptor are exposed at the surface of the transformed cellular host,
- the placing of this cellular host in contact with these determined ligands,
- the detection of an affinity reaction between the said transformed cellular host and the said determined ligands.

18. Kit for detecting the possible affinity of a ligand for a polypeptide according to any one of Claims 1 to 4, comprising:
- a culture of cellular hosts transformed by a modified vector as defined in any one of Claims 10 to 12 or a culture of cellular hosts according to Claim 12,
- physical or chemical means for inducing the expression of the nucleotide sequence contained in the modified vector when the promoter placed upstream of this sequence is a promoter inducible by the said physical or chemical means, and obtaining a protein,
- one or more control ligands having determined affinities for the abovementioned polypeptide,
- physical or chemical means for characterizing the biological activity of the expressed protein.

## Patentansprüche

1. Polypeptid mit einer Aktivität des adrenergischen β-Rezeptors, enthaltend:
die Sequenz von 402 Aminosäuren gemäß Figur 1, oder ein Fragment dieser Sequenz, wobei das Fragment
trotzdem die in dieser Sequenz enthaltenen Stellen aufweist und dessen Gegenwart notwendig ist, so daß, wenn dieses Fragment an der Zelloberfläche exponiert ist, es fähig ist, an der Aktivierung der Adenylatcyclase in Gegenwart eines Agonisten mitzuwirken, wobei diese Aktivierung in der Reihenfolge der folgenden Agonisten ansteigt: Salbutamol, BRL 28410, BRL 37344 und (1)-Isoproterenol, oder
von den Antikörpern erkannt werden kann, die die Sequenz von 402 Aminosäuren ebenfalls erkennen, aber weder den adrenergischen β1-Rezeptornoch den adrenergischen β2-Rezeptor, oder
zur Erzeugung von Antikörpern fähig ist, die die Sequenz von 402 Aminosäuren erkennen, aber weder den adrenergischen β1-Rezeptor noch den adrenergischen β2-Rezeptor.

2. Polypeptid nach Anspruch 1, dessen Fähigkeit zur Stimulation der Adenylatcyclase und/oder der Anhäufung von cAMP, die durch Isoproterenol induziert wird, durch die folgenden Verbindungen nicht gehemmt wird:
- Practolol,
- Nadolol,
- CGP 12,177,
- Butoxamin,
- Alprenolol,
- Propanolol,
- Pindolol,
- Oxprenolol,
wenn diese bei Konzentrationen unter oder in Höhe von 10⁻⁴ M unter folgenden Bedingungen verwendet werden:
zur Anhäufung von cyclischem AMP werden die Zellen gezüchtet und nach Behandlung mit Versen/EDTA (Eurobio, Paris) gewonnen, gewaschen und in Hank-Medium resuspendiert, das 20 mM Hepespuffer, pH 7,4, 1 mM Ascorbinsäure und 0,1 mM Isobutylmethylxanthin enthält, Aliquots von 10⁶ Zellen werden 30 Minuten mit Inhibitoren von 10⁻⁴ M inkubiert, vor Zugabe von 5x10⁻⁹ M Isoproterenol, die Inkubation wird weitere 30 Minuten fortgesetzt und der Gehalt an cyclischem AMP gemessen, beispielsweise gemäß den Vorschriften des Testkits von Amersham.

3. Polypeptid nach Anspruch 1, dadurch gekennzeichnet, daß Pindolol und Oxprenolol die Adenylatcyclase und/oder die Anhäufung von cyclischem AMP in den mit dem Polypeptid transfizierten CHO-Zellen unter folgenden Bedingungen stimulieren:
zur Anhäufung von cyclischem AMP werden die Zellen gezüchtet und nach Behandlung mit Versen/EDTA (Eurobio, Paris) gewonnen, gewaschen und in Hank-Medium resuspendiert, das 20 mM Hepespuffer, pH 7,4, 1 mM Ascorbinsäure und 0,1 mM Isobutylmethylxanthin enthält, Aliquots von 10⁶ Zellen werden 30 Minuten in einem Gesamtvolumen von 1 ml mit Konzentrationen an Pindolol oder Oxprenolol, die höher sind als 10⁻¹¹ M, inkubiert und der cAMP-Gehalt gemessen, zum Beispiel gemäß der Vorschriften des Testkits von Amersham.

4. Polypeptid nach Anspruch 1, dadurch gekennzeichnet, daß es aus der Sequenz gemäß Figur 1 besteht, die sich von dem Ende, das die Aminosäure in Position 1 ausmacht, bis zu dem Ende, das die Aminosäure in Position 402 ausmacht, erstreckt.

5. Nucleinsäure, dadurch gekennzeichnet, daß sie eine Kette von Nucleotiden, die die Polypeptide nach den Ansprüchen 1 bis 4 codieren, enthält oder daraus besteht.

6. Nucleinsäure nach Anspruch 5, dadurch gekennzeichnet, daß sie die Kette von Nucleotiden gemäß der Figur 1 bis enthält oder daraus besteht, die sich von dem Ende, das aus Nucleotid 1 besteht, bis zu dem Ende, das aus Nucleotid 2022 besteht, erstreckt.

7. Rekombinanter Vektor, insbesondere für die Clonierung und/oder Expression, insbesondere ein Plasmid, Cosmid oder Phage, dadurch gekennzeichnet, daß er eine Nucleinsäure nach einem der Ansprüche 5 und 6 an einer der Stellen enthält, die nicht essentiell für seine Replikation sind.

8. Rekombinanter Vektor nach Anspruch 7, dadurch gekennzeichnet, daß sie sich um den Vektor M13mp18-Huβ3, (Nr. 1085) handelt, bestehend aus einem Bakteriophagen, der unter der Nummer I-833 am 20. Januar 1989 bei CNCM, 25 rue de Docteur Roux, Paris, hinterlegt wurde.

9. Rekombinanter Vektor nach Anspruch 7, dadurch gekennzeichnet, daß er an einer der Stellen, die nicht für seine Replikation essentiell sind, für die Förderung der Expression einer Aminosäuresequenz nach den Ansprüchen 1-4 in einem zellulären Wirt notwendige Elemente und gegebenenfalls einen Promotor, der von den Polymerasen des zellulären Wirts erkannt wird, insbesondere einen induzierbaren Promotor, und gegebenenfalls eine Signalsequenz und eine Verankerungssequenz enthält.

10. Zellulärer Wirt, der mit einem rekombinanten Vektor nach einem der Ansprüche 7 bis 9 transformiert ist und Regulationselemente enthält, die die Expression der Nucleotidsequenz, die das Polypeptid nach einem der Ansprüche 1-4 codiert, in dem Wirt erlauben.

11. Transformierter zellulärer Wirt nach Anspruch 10, dadurch gekennzeichnet, daß er aus Bakterien, insbesondere E. coli, ausgewählt ist.

12. Transformierter zellulärer Wirt nach Anspruch 10, dadurch gekennzeichnet, daß er aus eukaryontischen Organismen, wie CHO-Zellen, ausgewählt ist.

13. Antikörper, dadurch gekennzeichnet, daß er (sie) spezifisch gegen ein Polypeptid nach einem der Ansprüche 1-4 gerichtet ist (sind) und daß er (sie) weder den adrenergischen β1-Rezeptor noch den adrenergischen β2-Rezeptor erkennt (erkennen) , und daß er (sie) insbesondere die folgenden Aminosäuresequenzen erkennt (erkennen):
1 bis 36 178 bis 201
64 bis 74 223 bis 291
101 bis 108 314 bis 324
133 bis 135 345 bis 402

14. Nucleotidsonde, dadurch gekennzeichnet, daß sie mit einer der Nucleinsäuren nach den Ansprüchen 5 und 6 oder deren komplementären Sequenzen unter Hybridisierungsbedingungen hybridisieren, bei denen sie nicht mit den Genen oder der mRNA der adrenergischen β1- oder β2-Rezeptoren hybridisieren, insbesondere die Sonden, die unter den folgenden Nucleotidsonden ausgewählt sind:
definiert durch die Nucleinsäuresequenz gemäß der Figur 1 bis, die sich von dem Ende, das aus dem Nucleotid in Position 1 besteht, bis zu dem Ende, das aus dem Nucleotid in Position 637 besteht, erstreckt,
definiert durch die Nucleinsäuresequenz gemäß Figur 1 bis, die sich von dem Ende, das aus dem Nucleotid in Position 638 besteht, bis zu dem Ende, das aus dem Nucleotid in Position 745 besteht, erstreckt,
definiert durch die Nucleinsäuresequenz gemäß Figur 1 bis, die sich von dem Ende, das aus dem Nucleotid in Position 1313 besteht, bis zu dem Ende, das aus dem Nucleotid in Position 1513 besteht, erstreckt,
definiert durch die Nucleinsäuresequenz gemäß Figur 1 bis, die sich von dem Ende, das aus dem Nucleotid in Position 1679 besteht, bis zu dem Ende, das aus dem Nucleotid in Position 1843 besteht, erstreckt,
definiert durch die Nucleinsäuresequenz gemäß Figur 1 bis, die sich von dem Ende, das aus dem Nucleotid in Position 1844 besteht, bis zu dem Ende, das aus dem Nucleotid in Position 2022 besteht, erstreckt,
definiert durch die Nucleinsäuresequenz gemäß Figur 1 bis, die sich von dem Ende, das aus dem Nucleotid in Position 638 besteht, bis zu dem Ende, das aus dem Nucleotid in Position 1843 besteht, erstreckt,
oder deren komplementäre Nucleotidsequenz.

15. Verfahren zur Herstellung eines Polypeptids nach einem der Ansprüche 1-4, dadurch gekennzeichnet, daß man in einem geeigneten Medium einen zellulären Wirt züchtet, der vorher mit einem rekombinanten Vektor transformiert wurde, welcher eine Nucleinsäure nach einem der Ansprüche 7-9 enthält, und aus der Kultur das von dem transformierten zellulären Wirt produzierte Polypeptid gewinnt.

16. Verfahren zum Nachweis der Fähigkeit eines Moleküls sich wie ein Ligand gegenüber einem Polypeptid nach einem der Ansprüche 1-4 zu verhalten, gekennzeichnet durch:
Inkontaktbringen des Moleküls mit einem zellulären Wirt, der vorher mit einem Vektor transformiert wurde, welcher selbst durch eine Insertion modifiziert wurde, die das Polypeptid codiert, wobei der Wirt auf seiner Oberfläche eine oder mehrere spezifische Stellen des Polypeptids trägt, gegebenenfalls nach Induktion der Expression dieser Insertion, wobei das Inkontaktbringen unter Bedingungen erfolgt, die die Ausbildung einer Bindung zwischen mindestens einer der spezifischen Stellen und dem Molekül ermöglicht, wenn es sich erweist, daß es eine wirksame Affinität für das Polypeptid besitzt,
Nachweis einer möglichen Bildung eines Komplexes von der Art Ligand-Polypeptid.

17. Verfahren zur Untersuchung der Affinität eines Polypeptids nach einem der Ansprüche 1-4 für einen oder mehrere bestimmte Liganden, gekennzeichnet durch:
Transformation eines kompetenten zellulären Wirts mit einem Vektor, insbesondere einem Plasmid oder einem Phagen, in den vorher eine Nucleotidsequenz inseriert wurde, die den β3-Rezeptor (Insertion) codiert, unter der Kontrolle von Regulationselementen, insbesondere einem Promotor, der von den Polymerasen des zellulären Wirts erkannt wird, und der die Expression der Nucleotidsequenz in dem verwendeten zellulären Wirt erlaubt,
die Züchtung des transformierten zellulären Wirts unter Bedingungen, die die Expression der Insertion und den Transport des exprimierten β3-Rezeptors zur Membran erlauben, so daß die Transmembransequenzen des β3- Rezeptors an der Oberfläche des transformierten zellulären Wirts exponiert sind,
Inkontaktbringen des zellulären Wirts mit den bestimmten Liganden,
Nachweis einer Affinitätsreaktion zwischen dem transformierten zellulären Wirt und den bestimmten Liganden.

18. Kit zum Nachweis der möglichen Affinität eines Liganden für ein Polypeptid nach einem der Ansprüche 1-4, umfassend:
eine Kultur von zellulären Wirten, die mit einem Vektor transformiert wurden, der gemäß einem der Ansprüche 10-12 modifiziert wurde, oder eine Kultur von zellulären Wirten nach Anspruch 12,
physikalische oder chemische Mittel zur Induktion der Expression der Nucleotidsequenzen, die in dem modifizierten Vektor enthalten sind, wenn der Promotor, der stromaufwärts von dieser Sequenz gelegen ist, ein durch diese physikalischen oder chemischen Mittel induzierbarer Promotor ist, und Gewinnung eines Proteins,
einen oder mehrere Kontrolliganden, die bestimmte Affinitäten für das Polypeptid aufweisen,
physikalische oder chemische Mittel für die Charakterisierung der biologischen Aktivität des exprimierten Proteins.
